# FASCICULE DE BREVET EUROPEEN

(11) **EP 0 509 466 B1**
(45) Date de publication et mention de la délivrance du brevet: **07.01.1998**
(21) Numéro de dépôt: 92106469.7
(22) Date de dépôt: 15.04.1992
(51) Int. Cl.: H05K 1/18

(54) **Dispositif d'interruption irréversible du fonctionnement d'un appareil commandé par un système électronique**
Vorrichtung zur irreversiblen Funktionsunterbrechung eines von einem elektronisches System gesteuerten Apparates
Device for irreversible functioning interruption of an apparatus controlled by an electronic system

(30) Priorité: 19.04.1991 FR 9104966
(43) Date de publication de la demande: 21.10.1992
(73) Titulaire: SMH Management Services AG, CH-2501 Biel (CH)
(72) Inventeur: Aubert, Christophe, CH-2052 Fontainemelon (CH)
(74) Mandataire: de Raemy, Jacques

(56) Documents cités:
- EP-A- 0 065 826
- EP-A- 0 447 909
- DE-A- 2 949 914
- FR-A- 1 582 980

## Description

La présente invention concerne un appareil médical comprenant un dispositif d'interruption irréversible de son fonctionnement, par exemple une pompe médicale du type péristaltique.

Les pompes médicales sont connues depuis plusieurs années et permettent d'administrer à un patient des médicaments à petites doses et notamment en continu. Ces pompes sont de petites dimensions et sont prévues pour être portées par le patient et pour avoir une autonomie de plusieurs jours, ce qui permet à celui-ci de ne plus être alité et de circuler librement. Ces pompes permettent notamment au patient de vivre chez lui sans être en permanence sous surveillance médicale.

L'absence du personnel médical surveillant ces patients, et le bon fonctionnement de leurs pompes, a conduit les autorités de la santé à exiger que ces pompes soient munies de nombreux systèmes de sécurité.

Ainsi, ces pompes sont généralement programmées à l'avance pour s'arrêter de fonctionner au bout d'un temps donné et de plus, tout mauvais fonctionnement de la pompe, tel que l'obstruction d'une canalisation ou l'apparition d'une surpression, est détectée et entraîne l'arrêt immédiat de la pompe.

Le document EP 0 447 909, publié le 25 septembre 1991 et déposé le 11 mars 1991, constitue un droit européen antérieur au sens de l'article CBE 54 (3 et 4). Ce document décrit une pompe portable d'administration d'une substance thérapeutique liquide comprenant, dans un mode de réalisation particulier, des moyens d'interruption de la marche de la pompe péristaltique actionnés par le désassemblage de deux modules principaux de la pompe. Selon l'enseignement de ce document, il est donc nécessaire de manipuler la pompe pour séparer les deux modules principaux de cette dernière, en brisant notamment un crochet muni de deux contacts électriques assurant une fonction d'interrupteur du circuit électrique de commande de cette pompe.

Les moyens d'interruption prévus dans le document susmentionné servent uniquement à assurer un usage unique de la pompe. En effet, cette pompe est une pompe jetable à usage unique et les moyens d'interruption servent à empêcher une utilisation ultérieure de la pompe, notamment après avoir rempli à nouveau le réservoir de cette pompe d'une substance quelconque.

Toutefois, un utilisateur de la pompe désirant, pour une raison ou une autre, arrêter le fonctionnement de cette pompe ne peut guère s'accommoder du dispositif d'interruption susmentionné pour stopper l'administration d'un médicament à laquelle il est soumis. En effet, le malade porteur de la pompe peut se sentir indisposé à un instant et souhaiter arrêter sa pompe, même si celle-ci fonctionne parfaitement correctement.

Il n'est toutefois pas envisageable de prévoir un simple interrupteur marche/arrêt, car alors le malade après avoir arrêté la pompe, pourrait remettre celle-ci en marche quand bon lui semble, sans suivre la posologie et la séquence d'administration du médicament prévues par le médecin. Ceci serait contraire aux normes de sécurité imposées et aux exigences du corps médical et c'est pourquoi il est nécessaire d'avoir des moyens d'interruption irréversible du fonctionnement de la pompe.

L'invention a pour but de répondre à ces impératifs.

A cet effet l'invention concerne un appareil médical comprenant un dispositif d'interruption irréversible tel que défini dans la revendication 1, dans lequel il est prévu un élément d'interruption mobile entre une position de repos autorisant le fonctionnement de l'appareil et une position d'interruption dans laquelle il entraîne l'interruption irréversible du fonctionnement de l'appareil en brisant un organe essentiel d'un système électronique de commande de l'appareil.

Dans le mode de réalisation préféré de l'invention, le système électronique comprend un circuit imprimé incorporant ledit organe essentiel.

Selon d'autres caractéristiques de l'invention, l'élément d'interruption comprend un piston coulissant à l'intérieur d'un orifice débouchant délimité par un carter, ce piston comprenant un corps central, une extrémité de rupture placée au voisinage dudit circuit imprimé et une tête de piston à laquelle l'utilisateur a accès et sur laquelle cet utilisateur peut agir pour déplacer ledit piston de la position de repos à la position d'interruption. En outre, l'extrémité de rupture est munie d'une partie formant saillie conçue pour rompre ledit circuit imprimé lorsque le piston est déplacé en position de rupture.

Ce dispositif est donc extrêmement simple à utiliser par un utilisateur non averti, qui a juste à exercer une pression sur ledit piston pour déplacer celui-ci.

Enfin, la tête de piston est surmontée de moyens de protection empêchant l'accès à ladite tête de piston, et présentant une zone de moindre résistance qui peut être rompue pour autoriser l'accès à la tête de piston.

Grâce à cette caractéristique, les moyens d'interruption ne peuvent pas être déclenchés de façon involontaire par l'utilisateur. Ceci est particulièrement intéressant quant l'appareil est une pompe médicale que l'utilisateur porte en permanence sur lui et qui est en contact avec le corps.

L'invention sera mieux comprise à la lecture de la description suivante d'un mode de réalisation de l'invention donnée à titre d'exemple illustratif, cette description étant faite en relation avec les dessins joints dans lesquels :
- la figure 1 représente une vue en perspective d'un appareil en deux parties assemblées, muni du dispositif d'interruption selon l'invention,
- la figure 2 est une vue de dessus de l'appareil de la figure 1, les deux parties de cet appareil n'étant pas complètement assemblées,
- la figure 3 est une vue en coupe selon la ligne III-III de la figure 2, mais les deux parties de l'appareil étant assemblées, et le dispositif d'interruption selon l'invention étant en position de repos,
- la figure 4 est identique à la figure 3 sauf que le dispositif d'interruption est en position d'interruption, et
- la figure 5 représente une vue de dessus, partielle du circuit imprimé utilisé avec le dispositif d'interruption selon l'invention.

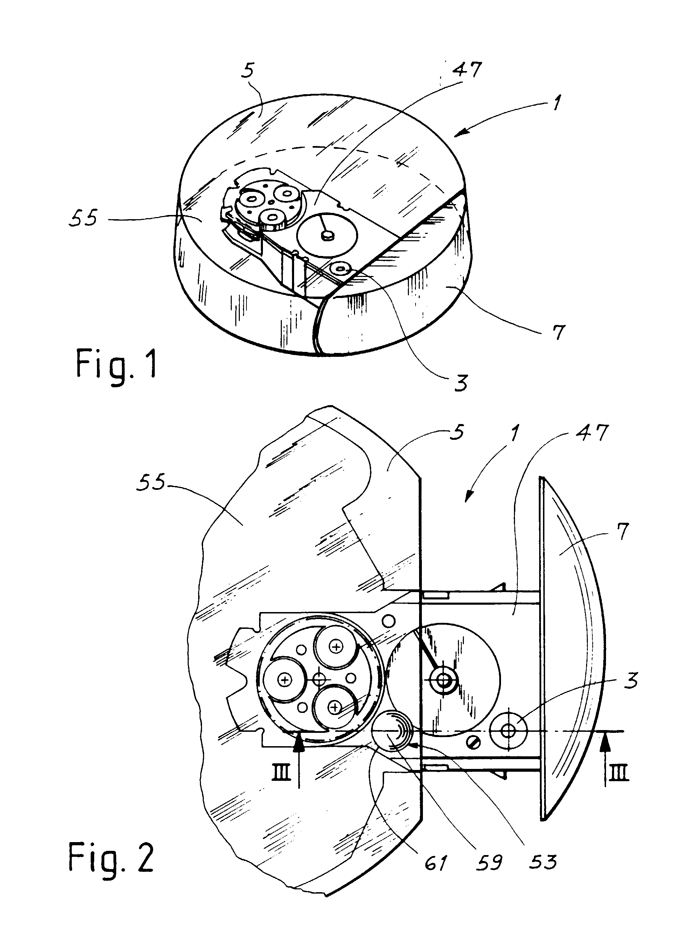

La figure 1, illustre un appareil 1, (il s'agit dans ce cas d'une pompe péristaltique médicale), muni du dispositif d'interruption irréversible 3 selon l'invention. Cet appareil 1 en deux parties comprend un module réservoir 5 et un module moteur 7. Un exemple de réalisation de cette pompe péristaltique est décrit dans la demande de brevet FR-90 03869. Toutefois, il est bien évident que cette pompe n'est pas un exemple limitatif du type d'appareil qui peut être muni du dispositif d'interruption selon l'invention. Le dispositif d'interruption peut être adapté dans n'importe quel appareil, notamment médical, nécessitant un dispositif d'arrêt d'urgence irréversible.

Comme illustré en figure 2, le module moteur 7 muni du dispositif d'interruption irréversible 3 selon l'invention, est engagé comme un tiroir à l'intérieur d'une cavité du module réservoir 5.

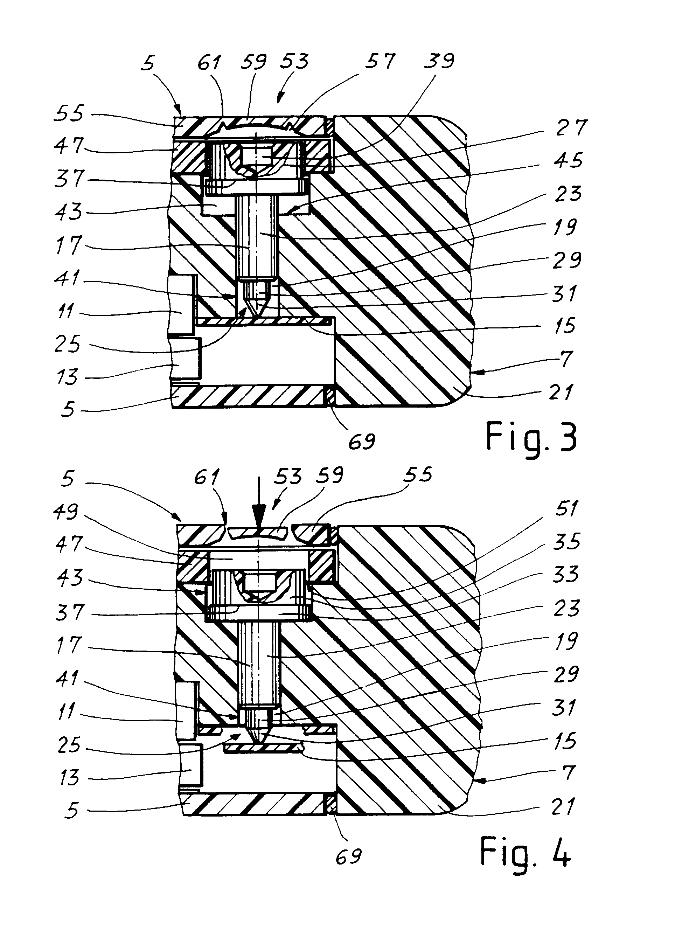

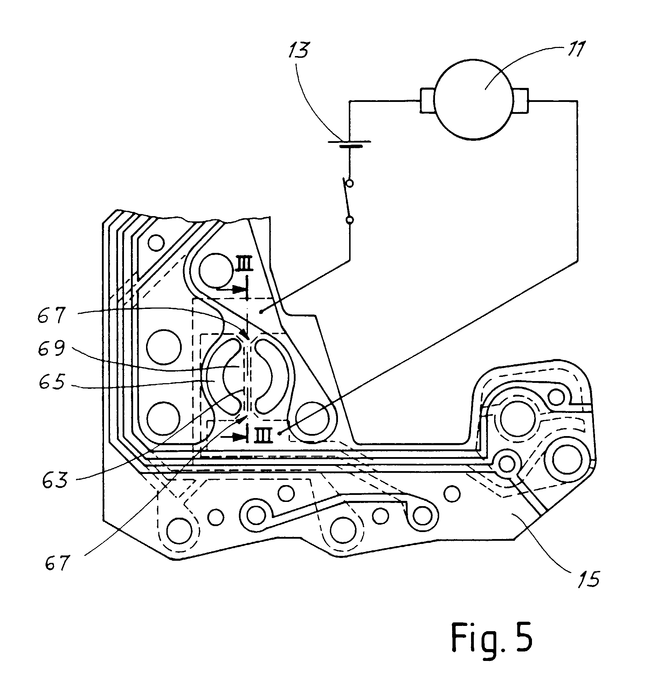

Le fonctionnement de l'appareil 1 se fait grâce à un moteur 11 alimenté par une source d'énergie 13 et commandé par un circuit imprimé 15, vus en coupe sur les figures 3 et 4.

Ce dispositif d'interruption 3 comprend au moins un élément d'interruption 17 mobile entre une première position de repos (illustrée en figure 3) autorisant le fonctionnement normal dudit appareil et une seconde position d'interruption (illustrée en figure 4) dans laquelle un organe essentiel dudit circuit imprimé 15 est brisé.

Plus précisément, l'élément d'interruption 17 a la forme d'un piston coulissant à l'intérieur d'un orifice débouchant 19, délimité par un carter 21 venu de matière avec le module moteur 7. Ce piston 17 comprend un corps central 23, une extrémité de rupture 25 placée au voisinage dudit circuit imprimé 15 et une tête de piston 27. De préférence, le corps central 23 et l'orifice débouchant 19 sont cylindriques.

L'extrémité de rupture 25 comprend une partie cylindrique 29 d'un diamètre inférieur à celui du corps central 23, cette partie 29 se terminant par une partie formant saillie 31 conçue pour briser un organe essentiel du circuit imprimé 15. Dans le mode de réalisation représenté ici, cette partie formant saillie 31 est sensiblement conique. Toutefois, elle pourrait également avoir toute autre forme de préférence pointue, permettant la rupture d'un organe essentiel dudit circuit imprimé, lorsque le piston 17 est déplacé.

La tête de piston 27 comprend une partie inférieure 33 reliée au corps central 23 et une partie supérieure 35 d'un diamètre inférieur à la partie 33, définissant avec cette dernière un épaulement annulaire 37. En outre, la partie supérieure 35 présente sur sa surface d'extrémité libre une cavité 39 conçue pour recevoir un outil permettant de faire coulisser le piston 17. Cette cavité 39 est coaxiale avec le piston 17.

Le piston 17 et l'orifice 19 débouchant au niveau du circuit imprimé 5, sont disposés selon un axe sensiblement perpendiculaire au plan dudit circuit imprimé.

L'orifice débouchant 19 comprend successivement depuis le circuit imprimé 15, jusque vers l'extérieur, une partie de coulissement allongée 41 d'un diamètre très légèrement supérieur à celui du corps de piston 23 et une chambre 43 d'un diamètre très légèrement supérieur à la partie inférieure 33 de la tête de piston 27. Ces légères différences de diamètre permettent le coulissement du piston 17.

La chambre 43 est d'un diamètre supérieur à celui de la partie de coulissement 41 et définit ainsi un épaulement annulaire 45.

Lors du montage, on introduit le piston 17 à l'intérieur de l'orifice débouchant 19 et l'on fixe au-dessus de cet orifice une plaque 47 présentant une ouverture circulaire 49 coaxiale avec l'orifice 19. Le diamètre de cette ouverture 49 est légèrement supérieur au diamètre de la partie supérieure 35 de la tête de piston 27 et ainsi cette partie 35 peut coulisser librement dans cette ouverture 49.

En outre, le diamètre de cette ouverture 49 est légèrement inférieur à celui de la chambre 43 et définit avec celle-ci une butée annulaire 51.

Selon une variante de réalisation, le dispositif d'interruption selon l'invention peut également comprendre des moyens de protection 53 de la tête de piston 27. Dans le cas de l'appareil représenté en figures 1 et 2, ces moyens de protection 53 seront prévus dans la face supérieure 55 du module réservoir 5 réalisé en matière plastique. Ces moyens de protection 53 sont conçus pour être placés juste au-dessus de la tête de piston 27 lorsque le module moteur 7 est engagé à l'intérieur du module réservoir 5.

Toutefois, lorsque l'appareil muni du dispositif d'interruption selon l'invention est en une seule partie, on peut prévoir lors du montage une dernière étape, au cours de laquelle on dispose au-dessus de la tête de piston 27 et de la plaque 47, une plaque de matière plastique formant les moyens de protection.

La surface inférieure de la face supérieure 55 du module réservoir 5 présente une gorge 57 délimitant une zone centrale circulaire 59 et définissant une zone 61 de moindre résistance.

La figure 5 illustre un mode de réalisation du circuit imprimé 15 partiellement représenté.

Ce circuit imprimé 15 présente une pluralité de pistes 63 dont un exemple de configuration est illustré en figure 5.

Le circuit imprimé 15 selon l'invention présente deux évidements 65 en forme de haricot, disposés en vis-à-vis de part et d'autre d'une ou de plusieurs pistes 63 que l'on souhaite briser et qui sont essentielles au fonctionnement de l'appareil commandé par le circuit 15. En effet, ces pistes 63 permettent de relier le moteur 11 à son alimentation 13, comme cela est schématiquement représenté sur la figure 5.

Ces évidements 65 définissent entre eux deux zones 67 de moindre résistance et une aire sensiblement circulaire 69 traversée par lesdites pistes 63. Le piston 17 est disposé de façon à ce que son extrémité de rupture soit en regard de cette aire 69 et des zones 67 et plus précisément que la saillie 31 soit posée sensiblement au centre de l'aire 69.

Selon une autre variante de réalisation, on peut prévoir de disposer les évidements 65 non plus autour de pistes 63, mais autour d'un autre organe essentiel du circuit imprimé, permettant le fonctionnement de l'appareil 1.

Le fonctionnement du dispositif d'interruption selon l'invention va maintenant être décrit plus en détail.

Lorsque l'utilisateur d'un appareil muni d'un dispositif d'interruption selon l'invention, par exemple un malade porteur d'une pompe médicale, souhaite arrêter le fonctionnement de ladite pompe, il utilise un objet pointu, tel qu'un crayon ou une punaise pour briser la zone circulaire 59 et enfoncer le piston 17. Celui-ci se déplace depuis la position de repos, illustrée en figure 3, jusqu'à la position d'interruption, illustrée en figure 4, où la saillie 31 dudit piston rompt une ou plusieurs pistes 63 du circuit imprimé 15. Cette rupture est facilitée par les zones de moindre résistance 67.

La course du piston 17 est limitée par l'épaulement 45 qui sert de butée à la tête de piston 27.

De plus, on observe que dans la position de repos illustrée en figure 3, le piston repose sur le circuit imprimé 15. Toutefois, si en cours de manipulation, l'appareil est retourné par l'utilisateur de 180°, la partie inférieure 33 de la tête de piston est retenue par la butée annulaire 51 et le piston 17 ne risque pas de sortir de l'orifice débouchant 19.

Enfin, on notera que les moyens de protection 53 permettent également d'assurer l'étanchéité du dispositif d'interruption 3. En effet, on prévoit généralement un joint d'étanchéité 69 entre le module réservoir 5 et le module moteur 7. Ce joint 69 associé aux moyens de protection 53 permet d'éviter que de l'eau, par exemple, ne rentre dans l'orifice débouchant 19. Ceci est particulièrement utile dans le cas d'une pompe médicale que l'utilisateur continue de porter même en se lavant.

## Revendications

1. Appareil médical comprenant un système électronique de commande (15) et un dispositif d'interruption irréversible du fonctionnement de cet appareil, ledit dispositif d'interruption comprenant au moins un élément d'interruption (17) mobile, sucseptible d'être actionné par pression par un utilisateur depuis l'extérieur dudit appareil entre une position de repos autorisant le fonctionnement de cet appareil et une position d'interruption dans laquelle le fonctionnement de cet appareil est interrompu de manière irréversible par le brisement d'un organe essentiel dudit système électronique.

2. Appareil médical selon la revendication 1, ledit système électronique comprenant un circuit imprimé (15), ledit organe essentiel brisé par l'actionnement dudit élément d'interruption (17) étant formé au moins par une piste (63) dudit circuit imprimé, cette piste (63) assurant une liaison essentielle entre un moteur (11) assurant le fonctionnement de l'appareil et une source d'énergie (13) alimentant ce moteur.

3. Appareil médical selon la revendication 2, ledit élément d'interruption (17) comprenant un piston coulissant à l'intérieur d'un orifice débouchant (19), délimité par un carter (21), ce piston (17) comprenant un corps central (23), une extrémité de rupture (25) placée au voisinage dudit circuit imprimé (15) et une tête de piston (27) sur laquelle l'utilisateur peut agir pour déplacer ledit piston (17) de la position de repos à la position d'interruption.

4. Appareil médical selon la revendication 3, ladite extrémité de rupture (25) étant munie d'une partie formant saillie (31), conçue pour rompre l'organe essentiel dudit circuit imprimé (15) lorsque le piston (17) est déplacé en position d'interruption.

5. Appareil médical selon la revendication 4, la partie formant saillie (31) de l'extrémité de rupture (25) étant sensiblement conique.

6. Appareil médical selon l'une des revendications 3 à 5, la tête de piston (27) étant de dimensions supérieures au corps central (23), et l'orifice débouchant (19) s'élargissant à l'une de ses extrémités pour définir un épaulement (45) coopérant avec la tête de piston (27) afin de limiter la course dudit piston (17).

7. Appareil médical selon l'une des revendications 3 à 6, la tête de piston (27) présentant à son extrémité libre une cavité (39) conçue pour recevoir un outil permettant de faire coulisser ledit piston (17).

8. Appareil médical selon l'une des revendications 3 à 7, que la tête de piston (27) étant surmontée de moyens de protection (53) empêchant l'accès à ladite tête de piston et présentant une zone de moindre résistance (61) qui peut être rompue pour autoriser l'accès à la tête de piston.

9. Appareil médical selon l'une des revendications 2 à 8, ledit circuit imprimé (15) présentant une zone de moindre résistance (65, 67) prévue au moins autour dudit organe essentiel de ce circuit imprimé, l'élément d'interruption étant placé en regard de cet organe essentiel.

10. Appareil médical selon l'une des revendications précédentes, cet appareil médical étant une pompe péristaltique.

## Patentansprüche

1. Medizinisches Gerät mit einem elektronischen Steuersystem (15) und einer Vorrichtung zum irreversiblen Unterbrechen der Funktion des Geräts, welche Unterbrechungsvorrichtung mindestens ein bewegliches Unterbrechungselement (17) umfaßt, das von dem Benutzer durch Druck von außerhalb des Geräts zwischen einer die Funktion des Geräts zulassenden Ruheposition und einer Unterbrechungsposition betätigbar ist, in welcher die Funktion des Geräts in irreversibler Weise durch Zerstören eines für das elektronische System wesentlichen Organs unterbrochen wird.

2. Medizinisches Gerät nach Anspruch 1, bei dem das elektronische System einen gedruckten Schaltkreis 15 umfaßt, das durch Betätigen des Unterbrechungselements (17) zerstörte wesentliche Organ von mindestens einer Leiterbahn (63) des gedruckten Schaltkreises gebildet ist, und diese Leiterbahn (63) die wesentliche Verbindung zwischen einem die Funktion des Geräts sicherstellenden Motor (11) und einer diesen Motor speisenden Energiequelle (13) sicherstellt.

3. Medizinisches Gerät nach Anspruch 2, bei dem das Unterbrechungselement (17) einen im Innern einer von einem Gehäuse (21) begrenzten Durchgangsbohrung (19) gleitbeweglichen Kolben umfaßt, welcher Kolben (17) einen Zentralkorpus (23), ein Aufreißende (25) nahe dem gedruckten Schaltkreis (15) und einen Kolbenkopf (27) umfaßt, auf den der Benutzer einwirken kann, um den Kolben (17) aus der Ruheposition in die Unterbrechungsposition zu verlagern.

4. Medizinisches Gerät nach Anspruch 3, bei dem das Aufreißende (25) mit einer einen Vorsprung (31) bildenden Partie versehen ist, ausgebildet für das Aufreißen des wesentlichen Organs des gedruckten Schaltkreises (15), wenn der Kolben (17) in die Unterbrechungsposition verlagert ist.

5. Medizinisches Gerät nach Anspruch 4, bei dem die den Vorsprung (31) bildende Partie des Aufreißendes (25) im wesentlichen konisch ist.

6. Medizinisches Gerät nach einem der Ansprüche 3 bis 5, bei dem der Kolbenkopf (27) größere Abmessungen als der Zentralkorpus (23) aufweist und die Durchgangsbohrung (19) sich an einem ihrer Enden zur Definition einer Schulter (45) aufweitet, die mit dem Kolbenkopf (27) zur Begrenzung des Hubes des Kolbens (17) zusammenwirkt.

7. Medizinisches Gerät nach einem der Ansprüche 3 bis 6, bei dem der Kolbenkopf (27) an seinem freien Ende eine Einsenkung (39) aufweist, die für die Aufnahme eines das Gleiten des Kolbens (17) ermöglichenden Werkzeugs ausgebildet ist.

8. Medizinisches Gerät nach einem der Ansprüche 3 bis 7, bei dem der Kolbenkopf (27) von Schutzmitteln (53) abgedeckt ist, die den Zugang zu dem Kolbenkopf verhindern und eine Schwächungszone (61) aufweisen, die aufbrechbar ist, um den Zugang zu dem Kolbenkopf zu ermöglichen.

9. Medizinisches Gerät nach einem der Ansprüche 2 bis 8, bei dem der gedruckte Schaltkreis (15) eine Zone verringerter Festigkeit (65, 67) aufweist, die zumindest rings um das wesentliche Organ dieses gedruckten Schaltkreises herum vorgesehen ist, wobei das Unterbrechungselement gegenüber diesem wesentlichen Organ plaziert ist.

10. Medizinisches Gerät nach einem der vorangehenden Ansprüche, das als peristaltische Pumpe ausgebildet ist.

## Claims

1. Medical apparatus comprising an electronic control system (15) and an irreversible arrangement for interruption of the functioning of such apparatus, said interruption arrangement comprising at least one movable interruption element (17) so arranged as to be actuable by a user's action external to said apparatus, between a rest position permitting the functioning of said apparatus and an interruption position in which it brings about the irreversible interruption of the functioning of said apparatus by breaking an essential element of said electronic system.

2. Medical apparatus according to claim 2, said electronic system comprising a printed circuit (15), said essential element broken by the actuationg of said interruption element (17) being at least formed by a track (63) of said printed circuit, such track (63) assuring an essential connection between a motor (11) assuring the functioning of the apparatus and an energy source (13) feeding such motor.

3. Medical apparatus according to claim 2, said interruption element (17) comprising a piston sliding within an open-ended orifice (19) bounded by a casing (21), such piston (17) comprising a central body (23), a rupturing end (25) placed in the vicinity of said printed circuit (15) and a piston head (27) on which the user may act in order to displace said piston (17) from the rest position to the interruption position.

4. Medical apparatus according to claim 3, said rupturing end (25) being provided with a projecting portion (31) designed for breaking the essential element of said printed circuit (15) when the piston (17) is displaced into the interruption position.

5. Medical apparatus according to claim 4, the portion (31) projecting from the rupturing end (25) being substantially conical.

6. Medical apparatus according to any of claims 3 to 5, the piston head (27) being of dimensions greater than those of the central body (23), and the open-ended orifice (19) being enlarged at one of its ends in order to define a shoulder (45) cooperating with the piston head (27) in order to limit the travel of said piston (17).

7. Medical apparatus according to any of claims 3 to 6, the piston head (27) exhibiting at its free end a cavity (39) designed to accommodate a tool allowing sliding of said piston (17).

8. Medical apparatus according to any of claims 3 to 7, the piston head (27) being surmounted by protection means (53) preventing access to said piston head and exhibiting a zone (61) of lesser resistance which may be broken to enable access to the piston head.

9. Medical apparatus according to any of claims 2 to 8, said printed circuit (15) exhibiting at least one zone (65, 67) of lesser resistance provided at least around said essential element of such printed circuit, the interruption element being placed facing such essential element.

10. Medical apparatus according to any of the preceding claims, such medical apparatus being a portable peristaltic pump.
